# EUROPEAN PATENT SPECIFICATION

(11) **EP 2 856 164 B1**
(45) Date of publication and mention of the grant of the patent: **11.07.2018**
(21) Application number: 13796896.2
(22) Date of filing: 31.05.2013
(51) Int. Cl.: G01N 33/573, G01N 33/577, C07K 16/18

(54) **HIGH-THROUGHPUT EARLY DETECTION AND POINT-OF-CARE EARLY DETECTION OF PREGNANT WOMEN SUSCEPTIBLE TO DEVELOPING PREECLAMPSIA**
FRÜHERKENNUNG DER ANFÄLLIGKEIT FÜR PRÄEKLAMPSIE BEI SCHWANGEREN FRAUEN AM PFLEGEORT MIT HOHEM DURCHSATZ
DÉTECTION PRÉCOCE À HAUT RENDEMENT ET DÉTECTION PRÉCOCE SUR LE LIEU DE SOINS DE FEMMES ENCEINTES SUSCEPTIBLES DE DÉVELOPPER UNE PRÉÉCLAMPSIE

(30) Priority: 01.06.2012 US 201261654461 P; 01.06.2012 US 201261654464 P
(43) Date of publication of application: 08.04.2015
(73) Proprietor: MAYO FOUNDATION FOR MEDICAL EDUCATION AND RESEARCH, Rochester, MN 55905 (US)
(72) Inventor: GAROVIC, Vesna, D., Rochester, Minnesota 55902 (US)
(74) Representative: Kunz, Herbert
(86) International application number: PCT/US2013/043727
(87) International publication number: WO 2013/181612

(56) References cited:
- US-A1- 2009 068 683
- GAROVIC ET AL: "Urinary podocyte excretion as a marker for preeclampsia", AMERICAN JOURNAL OF OBSTETRICS & GYNECOLOGY, MOSBY, ST LOUIS, MO, US, vol. 196, no. 4, 31 March 2007 (2007-03-31), pages 320.e1-320.e7, XP022280068, ISSN: 0002-9378, DOI: 10.1016/J.AJOG.2007.02.007
- DURAN RAMSURAN ET AL: "The role of podocytes in the early detection of pre-eclampsia", CARDIOVASCULAR HEALTH, ELSEVIER, AMSTERDAM, NL, vol. 2, no. 1, 28 September 2011 (2011-09-28), pages 43-47, XP028393459, ISSN: 2210-7789, DOI: 10.1016/J.PREGHY.2011.09.004 [retrieved on 2011-10-12]
- GAROVIC VESNA ET AL: "Flow cytometry as a novel method for detection of podocyturia in preeclampsia", AMERICAN JOURNAL OF OBSTETRICS & GYNECOLOGY, MOSBY, ST LOUIS, MO, US, vol. 206, no. 1, Suppl, 1 January 2012 (2012-01-01), pages S349-S350, XP009187567, ISSN: 0002-9378
- FEDERICA COLLINO ET AL.: 'Preeclamptic sera induce nephrin shedding from podocytes through endothelin-1 release by endothelial glomerular cells' AM J PHYSIOL RENAL PHYSIOL vol. 294, no. 5, 2008, pages F1185 - F1194, XP055179773
- YUPING WANG ET AL.: 'Increased urinary excretion of nephrin, podocalyxin, and betaig-h3 in women with preeclampsia' AM J PHYSIOL RENAL PHYSIOL vol. 302, no. 9, 01 February 2012, pages F1084 - F1089, XP055179775
- MARCELLO CAMICI.: 'Urinary biomarkers of podocyte injury' BIOMARKERS MED. vol. 2, no. 6, 2008, pages 613 - 616, XP008175727

### SEQUENCE LISTING

<110> Mayo Foundation for Medical Education and Research
<120> HIGH-THROUGHPUT EARLY DETECTION AND POINT-OF-CARE
   EARLY DETECTION OF PREGNANT WOMEN SUSCEPTIBLE
   TO DEVELOPING PREECLAMPSIA
<130> 07039-1156WO1
<150> US 61/654,461
   <151> 2012-06-01
<150> US 61/654,464
   <151> 2012-06-01
<160> 3
<170> FastSEQ for Windows Version 4.0
<210> 1
   <211> 12
   <212> PRT
   <213> Homo sapiens
<400> 1
   Gln Glu Ala Gly Pro Glu Pro Ser Gly Ser Gly Arg
    1               5                   10
<210> 2
   <211> 22
   <212> PRT
   <213> Homo sapiens
<400> 2
   Gly Arg Gly Arg Gln Glu Ala Gly Pro Glu Pro Ser Gly Ser Gly Arg
    1               5                   10                  15
   Ala Gly Thr Pro Gly Cys
               20
<210> 3
   <211> 26
   <212> PRT
   <213> Homo sapiens
<400> 3
   Cys Gly Ser Leu Pro Phe Pro Ser Pro Ser Lys Pro Val Glu Pro Leu
    1               5                   10                  15
   Asn Pro Lys Lys Lys Asp Ser Pro Met Leu
               20                  25

## Description

### BACKGROUND

### 1. Technical Field

This document relates to methods and materials involved in high-throughput early detection of pregnant women susceptible to developing preeclampsia. For example, this document provides methods and materials related to performing flow cytometry to detect pregnant women who are susceptible to developing preeclampsia. This document also relates to methods and materials involved in point-of-care early detection of pregnant women susceptible to developing preeclampsia. For example, this document provides methods and materials related to performing an enzyme-linked immunosorbent assay (ELISA) to detect pregnant women who are susceptible to developing preeclampsia.

### 2. Background Information

Preeclampsia is a pregnancy-specific disease that affects about 5 percent of all pregnancies and remains a leading cause of both maternal and fetal morbidity and death worldwide. It is characterized by hypertension (blood pressure, ≥ 140/90 mm Hg) and proteinuria (≥ 300 mg in a 24-hour urine sample) that occur after 20 weeks of gestation. Proteinuria in preeclampsia is associated with characteristic renal pathologic changes of glomerular endotheliosis, which is considered to be a hallmark of preeclampsia in humans.

### SUMMARY

The present invention is as defined in the appended claims.

This document provides methods and materials related to high-throughput early detection of pregnant women susceptible to developing preeclampsia. For example, this document provides methods and materials related to performing flow cytometry to detect an elevated level of urinary podocytes within a urine sample of a pregnant woman early during her pregnancy to identify her as being susceptible to developing preeclampsia later during her pregnancy. In some cases, the flow cytometry techniques provided herein can be used at a laboratory in a high-throughput manner to detect an elevated level of urinary podocytes within a urine sample of a pregnant woman early during her pregnancy to identify her as being susceptible to developing preeclampsia later during her pregnancy.

This document also provides methods and materials related to performing flow cytometry to detect a lack of an elevated level of urinary podocytes within a urine sample of a pregnant woman early during her pregnancy to identify her as not being susceptible to developing preeclampsia later during her pregnancy. In some cases, the flow cytometry techniques provided herein can be used at a laboratory in a high-throughput manner to detect an absence of an elevated level of urinary podocytes within a urine sample of a pregnant woman early during her pregnancy to identify her as not being susceptible to developing preeclampsia later during her pregnancy.

Further, this document provides methods and materials related to point-of-care early detection of pregnant women susceptible to developing preeclampsia. For example, this document provides methods and materials related to performing an ELISA to detect an elevated level of urinary podocytes within a urine sample of a pregnant woman early during her pregnancy to identify her as being susceptible to developing preeclampsia later during her pregnancy. In some cases, an ELISA provided herein can be used at the point-of-care to detect an elevated level of urinary podocytes within a urine sample of a pregnant woman early during her pregnancy to identify her as being susceptible to developing preeclampsia later during her pregnancy.

In addition, this document provides methods and materials related to performing an ELISA to detect a lack of an elevated level of urinary podocytes within a urine sample of a pregnant woman early during her pregnancy to identify her as not being susceptible to developing preeclampsia later during her pregnancy. In some cases, an ELISA provided herein can be used at the point-of-care to detect an absence of an elevated level of urinary podocytes within a urine sample of a pregnant woman early during her pregnancy to identify her as not being susceptible to developing preeclampsia later during her pregnancy.

Identifying pregnant women who are susceptible to developing preeclampsia can allow such women, who are at risk for both maternal and fetal morbidity and death, to be monitored and treated timely for hypertension. In addition, identifying pregnant women who are not susceptible to developing preeclampsia can avoid unnecessary monitoring and treatment of the pregnant women.

In general, one aspect of this document features a flow cytometry method for the early detection of a pregnant woman susceptible to developing preeclampsia. The method comprises, or consists essentially of, (a) performing flow cytometry using a urine sample obtained from a pregnant woman, prior to development of preeclampsia and from gestational week 23 to gestational week 28, to detect the presence of an elevated level of urinary podocytes, and (b) classifying the pregnant woman as being susceptible to developing preeclampsia later during her pregnancy. The flow cytometry can comprise using an anti-podocin antibody. The flow cytometry can comprise using an anti-podocalyxin antibody. The flow cytometry can comprise using an anti-nephrin antibody. The flow cytometry can comprise using an anti-synaptopodin antibody. The urine sample can be a sample obtained from the pregnant woman from gestational week 24 to gestational week 28. The urine sample can be a sample obtained from the pregnant woman from gestational week 25 to gestational week 28. The urine sample can be a sample obtained from the pregnant woman from gestational week 26 to gestational week 28. The urine sample can be a sample obtained from the pregnant woman from gestational week 23 to gestational week 27. The urine sample can be a sample obtained from the pregnant woman from gestational week 23 to gestational week 26.

In another aspect, this document features a method for assessing a pregnant woman's susceptibility to developing preeclampsia later during her pregnancy. The method comprises, or consists essentially of, determining whether or not a urine sample obtained from the pregnant woman from gestational week 23 to gestational week 28 contains an elevated level of urinary podocytes, wherein the presence of the elevated level indicates that the pregnant woman is susceptible to developing preeclampsia later during her pregnancy, and wherein the absence of the elevated level indicates that the pregnant woman is not susceptible to developing preeclampsia later during her pregnancy. The determining step can comprise using an antibody to detect podocytes. The antibody can be an anti-podocin antibody. The antibody can be an anti-podocalyxin antibody. The antibody can be an anti-nephrin antibody. The antibody can be an anti-synaptopodin antibody. The method can comprise classifying the pregnant woman as being susceptible to developing preeclampsia later during her pregnancy if the elevated level is present, and classifying the pregnant woman as not being susceptible to developing preeclampsia later during her pregnancy if the elevated level is not present.

In another aspect, this document features an enzyme-linked immunosorbent assay method for the early detection of a pregnant woman susceptible to developing preeclampsia. The method comprises, or consists essentially of, (a) performing an enzyme-linked immunosorbent assay using a urine sample obtained from a pregnant woman, prior to development of preeclampsia and from gestational week 23 to gestational week 28, to detect the presence of an elevated level of urinary podocytes, and (b) classifying the pregnant woman as being susceptible to developing preeclampsia later during her pregnancy. The enzyme-linked immunosorbent assay can comprise using an anti-podocin antibody. The enzyme-linked immunosorbent assay can comprise using an anti-podocalyxin antibody. The enzyme-linked immunosorbent assay can comprise using an anti-nephrin antibody. The enzyme-linked immunosorbent assay can comprise using an anti-synaptopodin antibody. The urine sample can be a sample obtained from the pregnant woman from gestational week 24 to gestational week 28. The urine sample can be a sample obtained from the pregnant woman from gestational week 25 to gestational week 28. The urine sample can be a sample obtained from the pregnant woman from gestational week 26 to gestational week 28. The urine sample can be a sample obtained from the pregnant woman from gestational week 23 to gestational week 27. The urine sample can be a sample obtained from the pregnant woman from gestational week 23 to gestational week 26.

In another aspect, this document features a method for assessing a pregnant woman's susceptibility to developing preeclampsia later during her pregnancy. The method comprises, or consists essentially of, determining whether or not a urine sample obtained from the pregnant woman from gestational week 23 to gestational week 28 contains an elevated level of urinary podocytes, wherein the presence of the elevated level indicates that the pregnant woman is susceptible to developing preeclampsia later during her pregnancy, and wherein the absence of the elevated level indicates that the pregnant woman is not susceptible to developing preeclampsia later during her pregnancy. The determining step can comprise using an antibody to detect podocytes. The antibody can be an anti-podocin antibody. The antibody can be an anti-podocalyxin antibody. The antibody can be an anti-nephrin antibody. The antibody can be an anti-synaptopodin antibody. The method can comprise classifying the pregnant woman as being susceptible to developing preeclampsia later during her pregnancy if the elevated level is present, and classifying the pregnant woman as not being susceptible to developing preeclampsia later during her pregnancy if the elevated level is not present.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

Figure 1 is a flow chart used for patient recruitment.
Figure 2 contains graphs plotting the levels of angiogenic markers in normotensive (NT), gestational hypertension (gHTN), and preeclamptic (PE) pregnancies at 150-210 days of gestation.
Figures 3A-3D contain graphs plotting ROC curves for sFlt-1 (3A), PIGF (3B), soluble endoglin (3C), and podocyturia (3D).
Figure 4 is a graph plotting protein per creatinine and podocytes per creatinine at mid-gestation and delivery for 15 preeclamptic patients.
Figures 5A and 5B are LC-MS/MS chromatograms. MRM transition was monitored for the tryptic peptide from podocin for a digest of immortalized mouse podocyte cell line digests without (Control: Figure 5A) and with recombinant human podocin added (Spike: Figure 5B).
Figure 6 contains LC-MS/MS chromatograms. MRM transition was monitored for the tryptic peptide from podocin for a digest of fixed cells from a patient with preeclampsia on the top (with a calculated tryptic peptide concentration of 3.4 fmol/mg creatinine), and the peak from the internal standard on the bottom.
Figure 7 is a photograph of cell staining using a podocyturia assay. Hoechst nuclear stain (which stains blue) and podocin antibody followed with a secondary, FITC-labeled antibody (which stains green) were used.
Figure 8 contains LC-MS/MS chromatograms from fixed cells: normotensive pregnant patient (top), preeclamptic patient (bottom), with a calculated tryptic peptide concentration of 0.42 versus 5.4 fmol/mg creatinine, respectively.
Figure 9 is a photograph of RT-PCR results for the podocyte polypeptides, synaptopodin (lane A), podocin (lane B), and nephrin (lane C), in urine of a woman with preeclampsia.
Figure 10 is a photograph of RT-PCR results for podocin in urine of a woman with preeclampsia (lane A) compared to the urine of two normotensive pregnant women (lanes B and C).

### DETAILED DESCRIPTION

This document provides methods and materials related to high-throughput early detection of pregnant women susceptible to developing preeclampsia. For example, this document provides methods and materials related to performing flow cytometry to detect an elevated level of urinary podocytes within a urine sample of a pregnant woman early during her pregnancy to identify her as being susceptible to developing preeclampsia later during her pregnancy. In some cases, the flow cytometry techniques provided herein can be used at a reference laboratory in a high-throughput manner to detect elevated levels of urinary podocytes within tens to hundreds of different urine samples of pregnant women early during their pregnancies to identify them as being susceptible to developing preeclampsia later during their pregnancies.

This document also provides methods and materials related to performing flow cytometry to detect a lack of an elevated level of urinary podocytes within a urine sample of a pregnant woman early during her pregnancy to identify her as not being susceptible to developing preeclampsia later during her pregnancy. In some cases, the flow cytometry techniques provided herein can be used at a reference laboratory in a high-throughput manner to detect the absence of elevated levels of urinary podocytes within tens to hundreds of different urine samples of pregnant women early during their pregnancies to identify them as not being susceptible to developing preeclampsia later during their pregnancies.

In addition, this document provides methods and materials related to point-of-care early detection of pregnant women susceptible to developing preeclampsia. For example, this document provides methods and materials related to performing an ELISA to detect an elevated level of urinary podocytes within a urine sample of a pregnant woman early during her pregnancy to identify her as being susceptible to developing preeclampsia later during her pregnancy. In some cases, an ELISA provided herein can be used at the point-of-care to detect an elevated level of urinary podocytes within a urine sample of a pregnant woman early during her pregnancy to identify her as being susceptible to developing preeclampsia later during her pregnancy.

Further, this document provides methods and materials related to performing an ELISA to detect a lack of an elevated level of urinary podocytes within a urine sample of a pregnant woman early during her pregnancy to identify her as not being susceptible to developing preeclampsia later during her pregnancy. In some cases, an ELISA provided herein can be used at the point-of-care to detect an absence of an elevated level of urinary podocytes within a urine sample of a pregnant woman early during her pregnancy to identify her as not being susceptible to developing preeclampsia later during her pregnancy.

As disclosed herein, if the level of urinary podocytes detected at a gestational time from 23 weeks to 30 weeks (e.g., from 23 to 28 weeks, from 24 to 28 weeks, from 25 to 28 weeks, from 26 to 28 weeks, from 27 to 28 weeks, from 25 to 27 weeks, or from 25 to 26 weeks) is elevated in a pregnant woman, then the woman can be classified as being susceptible to developing preeclampsia later during her pregnancy (e.g., from about gestational week 28 to delivery, from about gestational week 29 to delivery, from about gestational week 30 to delivery, from about gestational week 31 to delivery, from about gestational week 32 to delivery, from about gestational week 33 to delivery, from about gestational week 34 to delivery, from about gestational week 35 to delivery, from about gestational week 36 to delivery, or from about gestational week 37 to delivery). If the level of urinary podocytes is not elevated in a pregnant woman at a gestational time from 23 weeks to 30 weeks (e.g., from 23 to 28 weeks, from 24 to 28 weeks, from 25 to 28 weeks, from 26 to 28 weeks, from 27 to 28 weeks, from 25 to 27 weeks, or from 25 to 26 weeks), then the woman can be classified as not being susceptible to developing preeclampsia later during her pregnancy (e.g., from about gestational week 28 to delivery, from about gestational week 29 to delivery, from about gestational week 30 to delivery, from about gestational week 31 to delivery, from about gestational week 32 to delivery, from about gestational week 33 to delivery, from about gestational week 34 to delivery, from about gestational week 35 to delivery, from about gestational week 36 to delivery, or from about gestational week 37 to delivery).

The methods and materials provided herein can be used to assess any pregnant woman for preeclampsia. In some cases, a woman pregnant for 23 weeks to 33 weeks (e.g., for 23 to 28 weeks, for 24 to 28 weeks, for 25 to 28 weeks, for 26 to 28 weeks, for 27 to 28 weeks, for 25 to 27 weeks, or for 25 to 26 weeks) can be assessed.

As described herein, flow cytometry can be used to determine the level of podocytes in a pregnant woman's urine. For example, flow cytometry that includes using antibodies that bind to podocytes or polypeptides expressed by podocytes can be used to determine the level of podocytes in a pregnant woman's urine. Examples of such antibodies for use with a flow cytometry technique provided herein include, without limitation, antibodies that have the ability to bind podocin, podocalyxin, nephrin, synaptopodin, Neph1, GLEPP1, WT1, CD2AP, actin, actinin, cadherin, catenin, integrin, vinculin, talin, paxillin, or ZO-1.

As described herein, an ELISA can be used to determine the level of podocytes in a pregnant woman's urine. For example, an ELISA that includes antibodies that bind to podocytes or polypeptides expressed by podocytes can be used to determine the level of podocytes in a pregnant woman's urine. Examples of such antibodies for use with an ELISA provided herein include, without limitation, antibodies that have the ability to bind podocin, podocalyxin, nephrin, synaptopodin, Neph1, GLEPP1, WT1, CD2AP, actin, actinin, cadherin, catenin, integrin, vinculin, talin, paxillin, or ZO-1.

The term "elevated level" as used herein with respect to the level of urinary podocytes is any level that is above a median urinary podocytes level in urine obtained between gestational weeks 30 and 36 from a random population of pregnant women (e.g., a random population of 10, 20, 30, 40, 50, 100, or 500 pregnant women) lacking preeclampsia throughout their pregnancies. In some cases, an elevated level of urinary podocytes as detected via flow cytometry can be a level that is greater than 25 urinary podocytes or podocin-positive cells (e.g., greater than 26, 27, 28, 29, 30, 35, 40, 45, 50, 75, 100, or more podocytes or podocin-positive cells) within a urine sample per mg of creatinine within a urine sample. In some cases, an elevated level of urinary podocytes can be a detectable level of podocin-positive cells within a urine sample. The presence or absence of such a detectable level of podocin-positive cells can be determined using flow cytometry that includes use of an anti-podocin antibody.

In some cases, an elevated level of urinary podocytes as detected via ELISA can be a level that is greater than 25 urinary podocytes or podocin-positive cells (e.g., greater than 26, 27, 28, 29, 30, 35, 40, 45, 50, 75, 100, or more podocytes or podocin-positive cells) within a urine sample per mg of creatinine within a urine sample. In some cases, an elevated level of urinary podocytes can be a detectable level of podocin-positive cells within a urine sample. The presence or absence of such a detectable level of podocin-positive cells can be determined using an ELISA that includes an anti-podocin antibody.

An antibody can be, without limitation, a polyclonal, monoclonal, human, humanized, chimeric, or single-chain antibody, or an antibody fragment having binding activity, such as a Fab fragment, F(ab') fragment, Fd fragment, fragment produced by a Fab expression library, fragment comprising a VL or VH domain, or epitope binding fragment of any of the above. An antibody can be of any type (e.g., IgG, IgM, IgD, IgA or IgY), class (e.g., IgG1, IgG4, or IgA2), or subclass. In addition, an antibody can be from any animal including birds and mammals. For example, an antibody can be a human, rabbit, sheep, or goat antibody. An antibody can be naturally occurring, recombinant, or synthetic. Antibodies can be generated and purified using any suitable methods known in the art. For example, monoclonal antibodies can be prepared using hybridoma, recombinant, or phage display technology, or a combination of such techniques. In some cases, antibody fragments can be produced synthetically or recombinantly from a gene encoding the partial antibody sequence. An anti-podocin antibody can bind to podocin polypeptides at an affinity of at least 10⁴ mol⁻¹ (e.g., at least 10⁵, 10⁶, 10⁷, 10⁸, 10⁹, 10¹⁰, 10¹¹, or 10¹² mol⁻¹).

In some cases, flow cytometry, ELISA, or mass spectrometry can be performed as described herein to detect an elevated level of urinary podocytes within a urine sample of a pregnant woman early during her pregnancy to identify her as being susceptible to developing preeclampsia later during her pregnancy. In some cases, flow cytometry, ELISA, or mass spectrometry can be performed as described herein to detect a lack of an elevated level of urinary podocytes within a urine sample of a pregnant woman early during her pregnancy to identify her as not being susceptible to developing preeclampsia later during her pregnancy.

Once the level of urinary podocytes of a pregnant woman is determined, then the level can be compared to a median level or a cutoff level and used to evaluate the pregnant woman for a susceptibility of developing preeclampsia later during her pregnancy. A level of urinary podocytes that is higher than the median level of urinary podocytes from a population of pregnant women having no preeclampsia (or a cutoff level) can indicate that the pregnant woman is susceptible to developing preeclampsia later during her pregnancy. A level of urinary podocytes that is lower than the median level of urinary podocytes from a population of pregnant women having no preeclampsia (or a cutoff level) can indicate that the pregnant woman is not susceptible to developing preeclampsia later during her pregnancy. A cutoff level can be set to any level provided that the values greater than that level are an increased level of urinary podocytes indicative of a pregnant woman being susceptible to developing preeclampsia. In some cases, a pregnant woman can be classified as being susceptible to developing preeclampsia later during her pregnancy if it is determined that the podocyte level in a urine sample from that woman is greater than the podocyte level in a urine sample obtained previously from that same woman.

A pregnant woman identified as being susceptible to developing preeclampsia later during her pregnancy as described herein can be monitored for the emergence of symptoms of preeclampsia (e.g., blood pressure ≥ 140/90 mm Hg and/or proteinuria such as ≥ 300 mg of protein in a 24-hour urine sample).

This document also provides methods and materials to assist medical or research professionals in determining whether or not a pregnant woman is susceptible to developing preeclampsia later during her pregnancy. Medical professionals can be, for example, doctors, nurses, medical laboratory technologists, and pharmacists. Research professionals can be, for example, principle investigators, research technicians, postdoctoral trainees, and graduate students. A professional can be assisted by (1) performing flow cytometry or an ELISA to detect an elevated level of urinary podocytes in a urine sample obtained from a pregnant woman between gestational weeks 23 and 33 (e.g., from gestational week 23 to 28, from gestational week 24 to 28, from gestational week 25 to 28, from gestational week 26 to 28, from gestational week 27 to 28, from gestational week 25 to 27, or from gestational week 25 to 26), and (2) communicating information about the presence of the elevated level to that professional.

Any appropriate method can be used to communicate information to another person (e.g., a professional). For example, information can be given directly or indirectly to a professional. In addition, any type of communication can be used to communicate the information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

The invention will be further described in the following examples, which do not limit the scope of the invention described in the claims.

### EXAMPLES

### Example 1 - Pregnant women susceptible to developing preeclampsia excrete urinary podocytes early during pregnancy

### Patient population

A longitudinal cohort study was conducted to examine the use of podocyturia, angiogenic, and anti-angiogenic factors in early and mid-pregnancy for the prediction of preeclampsia later in pregnancy. All participants gave written informed consent before inclusion in the study.

A total of 315 patients were prospectively enrolled at their first obstetric visit and followed until 4-8 weeks post-delivery. All study patients were included for only one pregnancy and were not re-enrolled for subsequent pregnancies. Blood and urine samples were collected at the first obstetrical visit (6-12 weeks), mid gestation (25-28 weeks), delivery, and again at 4-8 weeks post-partum.

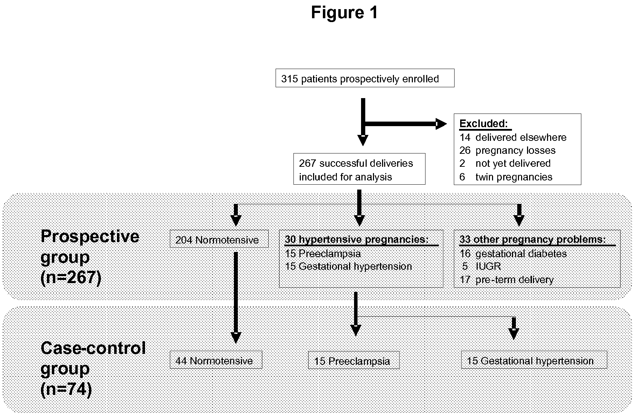

Of the 315 patients enrolled, there were 26 pregnancy losses. 14 were lost to follow-up or delivered elsewhere. As multiple gestations can have unpredictable effects on the levels of angiogenic factors, six twin pregnancies were excluded from analysis. Of the remaining 267 patients (Figure 1) who successfully delivered at the study location, 15 developed preeclampsia. Two of these patients, in addition to hypertension and proteinuria, demonstrated the characteristic findings of HELLP (hemolysis, elevated liver function tests, low platelet count) syndrome, which is currently categorized as a particularly severe form of preeclampsia. Fifteen patients developed gestational hypertension, and 16 developed gestational diabetes mellitus.

In order to study podocyturia in preeclamptic patients compared to those with normal pregnancies, three controls were selected for each of the 15 preeclamptic women (n=15). Controls were matched for maternal age and number of previous gestations. There were a total of 44 controls; one preeclamptic woman had only two controls due to a lack of appropriate age and gestation matched normal pregnancies. In order to assess podocyturia in women with gestational hypertension (without preeclampsia), data were also collected for the 15 women with this disorder.

At the time of enrollment, 38 pregnancies were identified as high risk for developing preeclampsia. This was based on either medical history and/or previous gestational history. Women with chronic kidney disease, diabetes mellitus, chronic hypertension, or systemic lupus erythematosus were considered high risk. Those with previous pregnancies affected by gestational hypertension, gestational diabetes, preeclampsia or eclampsia were also considered to be at high risk for the development of preeclampsia during the current pregnancy. Women were therefore classified as high risk for the later development of preeclampsia based on information available at the time of study enrollment.

### Serum studies

Serum samples were obtained at four time points during and after pregnancy. Samples were collected in SST tubes, centrifuged at 1500 X g, and stored at -80°C until the time of analysis. After thawing, samples were not refrozen. Serum concentrations of angiogenic factors (total sFlt-1, free PlGF, and soluble endoglin) were measured by Quantikine ELISA (enzyme-linked immunosorbent assay) kits (R&D Systems, Minneapolis, MN).

### Urine chemistries

Concurrent with serum collection, clean-catch urine specimens (50-100 mL) were obtained. The concentrations of albumin, total urinary protein, and creatinine were measured using standard methods on a Hitachi 911 Chemistry Analyzer (Roche Diagnostics, Indianapolis, IN).

### Podocyturia assay

Random urine samples (25-50 mL each) were centrifuged for eight minutes at 700 X g at room temperature. The pellets were rinsed twice with human diploid fibroblast (HDF) solution. Next, the pellets were re-suspended in Dulbecco's modified eagle's medium with 10% fetal bovine serum that was supplemented with penicillin, streptomycin, and fungisome for the prevention of contamination. One-milliliter aliquots were placed on collagen-coated tissue culture grade cover slips. Following overnight incubation at 37°C in 5% CO₂, the medium was removed, followed by two gentle washes in phosphate-buffered saline (PBS) solution. Cover slips were thoroughly dried and frozen at -80°C until the time of analysis.

### Immunofluorescence

Cover slips were placed immediately in -20°C methanol for 10 minutes, followed by two rinses in PBS for 5 minutes each, without agitation. Permeabilization was achieved by incubating for 10 minutes in 0.25% Triton-X at room temperature, followed by two rinses in PBS for 5 minutes each, without agitation.

Each cover slip was incubated with antibodies to podocin (Sigma Rabbit anti-Podocin #P0372-200 µLl) at a dilution of 1:50 overnight at 4°C. After two washes at room temperature with PBS for 5 minutes each, a fluorescein isothiocyanate (FITC) labeled goat anti-rabbit secondary antibody was added at a dilution of 1:50 for 1 hour at room temperature. This was followed by two room temperature PBS washes of 5 minutes each, without agitation. Coverslips were mounted using VectaShield mounting media with 4',6-diamidino-2-phenylindole (DAPI) nuclear stain to facilitate the differentiation of nucleated whole cells from cell fragments (Vector Labs, Burlington, CA). The stained slides were viewed with a fluorescence microscope (Leica, Germany). Nucleated cells staining for podocin were considered to be podocytes. A renal pathologist, who was blinded to the clinical diagnosis and laboratory findings, evaluated each sample to determine the number of cells that were present and the percentage of cells that were stained for podocyte markers. Podocyturia was expressed as a ratio of the number of podocytes to the creatinine content of the respective urine sample.

### Statistical Methods

The purpose of the statistical analysis was to assess the predictive value (separately, as well as incrementally) of clinical variables, angiogenic factors, and podocyturia, presence vs. absence, for the prediction of two endpoints: (1) a diagnosis of gestational hypertension (with or without preeclampsia) and (2) a diagnosis of preeclampsia.

Since these events occurred entirely after 210 days gestation, data known prior to gestational day 210 were elected to be considered as potentially predictive, and logistic models for the occurrence or non-occurrence of preeclampsia or gestational hypertension were fitted. These analyses were based on the 267 pregnancies that resulted in successful deliveries, excluding the 26 pregnancy losses and six twin pregnancies. For the assessment of podocyturia, the analysis was limited to the women for whom these data were available (15 with preeclampsia, 15 with gestational hypertension, and 44 normotensive, pregnant matched controls).

First, baseline clinical variables were assessed in the prediction of gestational hypertension and preeclampsia. After assessing all clinical variables univariately, multivariable logistic regression models were developed using a backward selection procedure, whereby variables were eliminated from the model until only those with a significance level of p<0.05 remained.

Next, the univariate and incremental values of angiogenic marker data were assessed. As these biomarkers change throughout pregnancy, it was attempted to normalize the data for gestational age by using the angiogenic marker data from all normal pregnancies. Specifically, for each angiogenic marker, a segmented linear model was fit in gestational age, both for the mean and for the scale parameter. Starting with potential knots at 40, 60, 80, 100, 160, 180, 220, 260, and 280 days gestation, the unnecessary knots were removed to arrive at a parsimonious model for each marker. The model for the mean was fit first, and the model for the scale was derived by fitting the same segmented linear model to the absolute residuals from the mean model. Finally, the mean and scale functions were used to transform the raw angiogenic data to Z-scores, defined as the residual of the raw angiogenic values from the mean function, divided by the appropriate multiple of the scale function, under a Gaussian assumption. These transformations, derived from normal pregnancies, were then applied to all values, not just those from normal pregnancies, to produce a Z-score for each angiogenic marker value.

The normalization of angiogenic factors involved fitting piecewise linear models to the mean and absolute deviations of the three angiogenic factors. The resulting transformations were applied to the pregnancies with events, as well as to the normal pregnancies, to yield Z-scores for each lab value for each pregnancy. For the purposes of predicting pregnancy outcomes, the last Z-score within the first 210 days of pregnancy was used.

Having constructed standardized Z-scores for each angiogenic marker, the most recent Z-score was used for each marker prior to 210 days as the analysis variable. Using logistic regression, the univariate predictive value, as well as the partial predictive value, was assessed for each of these markers after controlling for the clinical variable model derived earlier. Multivariable models were then constructed by including the Z-scores for the angiogenic markers in the multivariable models previously created using only clinical variables. A backward elimination procedure was again applied until only variables with a significance level of p<0.05 remained.

Finally, in order to assess the univariate and incremental predictive value of podocyturia, the clinical, angiogenic, and podocyturia data on 15 preeclampsia cases, their 44 matched controls, and the 15 women who experienced gestational hypertension were analyzed. Exact binomial confidence intervals and Fisher exact tests were used for the univariate effect of podocyturia on these endpoints. For the analysis of incremental value, the clinical/angiogenic models were imported from the first two stages of analysis, and the predictive value, with and without the inclusion of podocyturia was compared. As podocyturia had 100% sensitivity and specificity for PE/HELLP, which invalidates the Wald chi-square tests, the log likelihood ratio chi-square criterion was used to judge the incremental value of podocyturia.

### Demographic results

At the time of enrollment, of the 267 patients who completed the study, 38 (14.2%) were classified as high risk to develop preeclampsia, based on a history of chronic kidney disease, diabetes mellitus, chronic hypertension, or systemic lupus erythematosus, or with previous pregnancies affected by gestational hypertension, gestational diabetes, preeclampsia or eclampsia (Table 1). In the absence of these conditions, the patients were classified as being at an average risk for preeclampsia. Compared to the average risk group, women in the high risk group were older and more likely to be multiparous. At the time of delivery, and with respect to the outcome of their current pregnancies, they were less likely to have a normotensive, complication-free pregnancy course, as assessed at the time of delivery (p<.001) (Table 1). Based on the current pregnancy outcomes, with respect to hypertensive pregnancy disorders, the baseline characteristics among the entire study population are shown in Table 2. Systolic and diastolic blood pressures, protein/creatinine ratios, and the values of angiogenic markers (sFlt-1, PlGF, and endoglin), which were measured both in the second trimester of their pregnancies and at the time of delivery for the entire cohort, are presented in Table 3.

**Table 1. Stratification according to pre-pregnancy risk for preeclampsia at the time of enrollment. High risk pregnancies included those women with a history of chronic kidney disease, diabetes mellitus, chronic hypertension, or systemic lupus erythematosus, or with previous pregnancies affected by gestational hypertension, gestational diabetes, preeclampsia or eclampsia.**

| | **Average Risk (N=229)** | **High Risk (N=38)** | ***P*-Value*** |
|---|---|---|---|
| **Baseline Variables** | | | |
| Maternal Age (years), mean (s.d.) | 29.9 ± 4.6 | 32.0 ± 4.8 | 0.010 |
| Nulliparous, n (%) | 107 (47%) | 7(18%) | 0.001 |
| MAP at delivery, mm Hg, n (%) | 89.9 ± 8.6 | 94.9 ± 10.6 | 0.001 |

| **Outcomes** | | | |
|---|---|---|---|
| Normotensive, n (%) | 184 (80%) | 20 (53%) | <.001 |
| Gestational Hypertension, n (%) | 12 (5%) | 3 (8%) | |
| Other Pregnancy Problems, n (%) | 23 (10%) | 10 (26%) | |
| PE, n (%) | 10 (4%) | 5 (13%) | |

| | | | |
|---|---|---|---|
| *One-way ANOVA for continuous variables, Chi-square test for discrete variables MAP- mean arterial pressure PE- preeclampsia | | | |

**Table 2. Subject characteristics (baseline variables) grouped by pregnancy outcome.**

| **Variable** | **Normo-tensive (N=204)** | **Gestational HTN (N=15)** | **PE (N=15)** | **Other Pregnancy Problems** (N=33)** | ***P*-Value*** |
|---|---|---|---|---|---|
| Maternal Age (years), mean (s.d.) | 30.1 ± 4.6 | 30.0 ± 3.4 | 29.2 ± 6.4 | 31.3 ± 4.8 | 0.43 |
| Nulliparous, n (%) | 79 (39%) | 10 (67%) | 11 (73%) | 14 (42%) | 0.014 |
| MAP mid-gestation, mm Hg, mean (s.d.) | 76.4 ± 6.6 | 84.0 ± 7.4 | 84.2 ± 8.5 | 76.7 ± 5.9 | <.001 |
| MAP at delivery, mm Hg, mean (s.d.) | 88.4 ± 6.7 | 106.7 ± 4.1 | 109.2 ± 5.1 | 87.7 ± 7.1 | <.001 |
| BMI at 8-12 weeks, kg/m2, mean (s.d.) | 25.9 ± 5.8 | 27.0 ± 4.1 | 27.9 ± 7.4 | 29.8 ± 8.3 | 0.007 |
| Smokers, n (%) | 19 (9%) | 1 (7%) | 4 (27%) | 3 (9%) | 0.18 |
| High risk pregnancy, n (%) | 20 (10%) | 3 (20%) | 5 (33%) | 10 (30%) | 0.002 |

| | | | | | |
|---|---|---|---|---|---|
| *One-way ANOVA for continuous variables, Chi-square test for discrete variables ** Preterm delivery, intrauterine growth retardation and gestational diabetes HTN- hypertension PE- preeclampsia MAP- mean arterial pressure BMI- body mass index | | | | | |

**Table 3. Clinical characteristics, angiogenic marker values, and podocyturia measurements at mid-gestation and at delivery**

| Variable | Group | Median | <210 days IQR (25^{th}, 75^{th} percentiles) | Median | Delivery IQR (25^{th}, 75^{th} percentiles) |
|---|---|---|---|---|---|
| **Diagnostic Criteria** | | | | | |
| Systolic BP | PE (n=15) | 116 | 110,122 | 144 | 142, 146 |
| | gHTN (n=15) | 116 | 112,120 | 141 | 138,145 |
| | Normotensive (n=204) | 106 | 100,110 | 122 | 117,127 |
| Diastolic BP | PE (n=15) | 68 | 62,76 | 91 | 87,95 |
| | gHTN (n=15) | 68 | 62,74 | 90 | 84,92 |
| | Normotensive (n=204) | 60 | 58,66 | 72 | 68,76 |
| Protein/ | PE (n=15) | 0.05 | 0.04,0.07 | 0.78 | 0.16,1.21 |
| Creatinine ratio | gHTN (n=15) | 0.05 | 0.02,0.05 | 0.07 | 0.04,0.11 |
| | Normotensive (n=44) | 0.04 | 0.03,0.05 | 0.09 | 0.06,0.14 |

| **Angiogenic Factors** | | | | | |
|---|---|---|---|---|---|
| Endoglin (ng/mL) | PE (n=15) | 5.92 | 4.54, 8.98 | 22.45 | 11.79, 39.07 |
| | gHTN (n=15) | 5.83 | 5.33, 8.85 | 21.83 | 15.27, 39.63 |
| | Normotensive (n=204) | 5.78 | 4.67, 7.32 | 12.25 | 8.13, 19.79 |
| Endoglin z-score | PE (n=15) | -0.19 | -0.82, 1.12 | 0.55 | 0.26, 1.43 |
| | gHTN (n=15) | -0.16 | -0.45, 0.91 | 0.44 | -0.15, 1.88 |
| | Normotensive (n=204) | -0.16 | -0.73, 0.59 | -0.15 | -0.65, 0.53 |
| PlGF (pg/mL) | PE (n=15) | 305.33 | 152.06, 514.58 | 117.69 | 99.64, 234.21 |
| | gHTN (n=15) | 435.36 | 331.33, 658.77 | 167.50 | 152.89, 192.89 |
| | Normotensive (n=204) | 393.31 | 222.93, 629.52 | 183.16 | 98.94, 286.46 |
| PlGF z-score | PE (n=15) | -0.69 | -1.77, 0.12 | -0.90 | -1.11, -0.17 |
| | gHTN (n=15) | -0.15 | -0.57, 0.51 | -0.16 | -0.56, 0.17 |
| | Normotensive (n=204) | -0.02 | -0.55, 0.67 | -0.01 | -0.69, 0.56 |
| sFlt-1 (pg/mL) | PE (n=15) | 2382.4 | 1652.0, 4092.0 | 8686.6 | 5176.0, 13530.8 |
| | gHTN (n=15) | 2364.0 | 1326.7, 3402.4 | 14816.0 | 6766.8, 18400.0 |
| | Normotensive (n=204) | 1739.5 | 1155.6, 2589.7 | 5107.1 | 2737.2, 9403.5 |
| sFlt-1 z-score | PE (n=15) | 0.56 | -0.25, 1.30 | 0.74 | 0.14, 1.83 |
| | gHTN (n=15) | 0.34 | -0.55, 1.07 | 1.13 | 0.24, 1.41 |
| | Normotensive (n=204) | -0.05 | -0.68, 0.72 | 0.00 | -0.64, 0.72 |

| **Podocyturia** | | | | | |
|---|---|---|---|---|---|
| Number of podocytes/mg of creatinine | PE (n=15) | 0.28 | 0.11,0.80 | 0.77 | 0.28,1.78 |
| | gHTN (n= 15) | --- | --- | * | --- |
| | Normotensive (n=44) | --- | --- | --- | --- |

| | | | | | |
|---|---|---|---|---|---|
| *Podocytes were present for one GHTN patient; podocytes/creatinine = 0.04 PE- preeclampsia BP- blood pressure gHTN- gestational hypertension Cr- creatinine PlGF- placental growth factor sFlt-1 - soluble fms-like tyrosine kinase receptor-1 | | | | | |

### Podocyturia in the prediction of preeclampsia

In order to assess the value of podocyturia in the prediction of preeclampsia, urine samples were collected and analyzed on all 15 preeclampsia cases, 44 matched normal pregnancy controls, and all 15 gestational hypertension cases (Table 3).

Of the 15 patients who later went on to develop preeclampsia, 100% had podocyturia prior to 210 days gestation, compared with none of either 15 who went on to develop gestational hypertension, or the 44 normal patients in whom podocyturia was checked. Podocyturia demonstrated 100% sensitivity and specificity in distinguishing preeclampsia from both normotensive pregnancies and those with gestational hypertension, taken either individually or combined.

### Clinical factors and angiogenic markers in the prediction of preeclampsia

When baseline variables were assessed for their associations with the two primary endpoints of gestational hypertension + preeclampsia and preeclampsia only, the univariate correlates of the former were parity and "high risk" pregnancy status, while the correlates of the latter were gestations, parity, smoking and "high risk" status (Table 4). Multivariable clinical models adjusting for parity and high risk pregnancy were constructed for predicting gestational hypertension + preeclampsia vs. normotensive pregnancies. The model for predicting preeclampsia vs. normotensive + gestational hypertension pregnancies was adjusted for number of gestations, smoking, and high risk pregnancy. These models were used as the base models for the analysis of the incremental values of angiogenic biomarkers and podocyturia in the prediction of preeclampsia.

**Table 4. Univariate logistic model summary for clinical variables.**

| | **PE, gHTN vs NORMAL** | | | **PE vs gHTN, NORMAL** | | |
|---|---|---|---|---|---|---|
| **Effect** | **OR** | **95% CI** | **P value** | **OR** | **95% CI** | **P value** |
| Age | 0.97 | (0.89, 1.05) | 0.46 | 0.95 | (0.85, 1.07) | 0.39 |
| Gestations | 0.81 | (0.59, 1.12) | 0.20 | 0.37 | (0.17, 0.81) | 0.012 |
| Parity | 0.63 | (0.39, 1.01) | 0.056 | 0.38 | (0.16, 0.91) | 0.031 |
| BMI | 1.03 | (0.97, 1.09) | 0.38 | 1.03 | (0.96, 1.12) | 0.37 |
| Smoking | 2.08 | (0.72, 6.03) | 0.18 | 3.84 | (1.13, 13.14) | 0.032 |
| High Risk Pregnancy | 2.83 | (1.14, 6.99) | 0.025 | 3.69 | (1.18, 11.55) | 0.025 |

| | | | | | | |
|---|---|---|---|---|---|---|
| PE- preeclampsia gHTN- gestational hypertension BMI- body mass index | | | | | | |

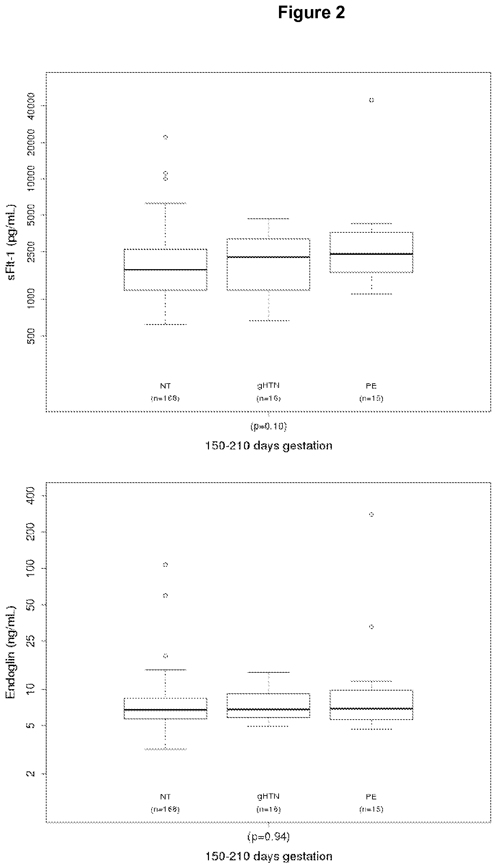

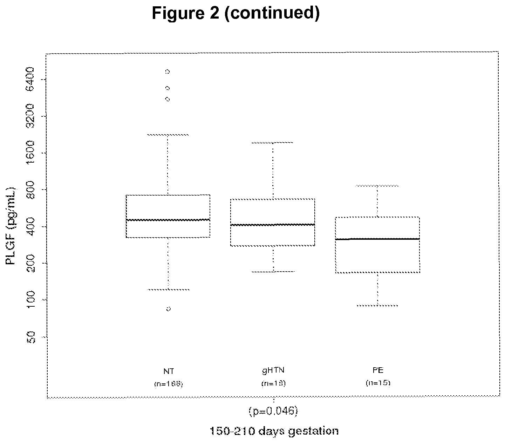

For each angiogenic marker, median values were compared among normotensive, gestational hypertension, and preeclamptic pregnancies measured at 150-210 days of gestation (Figure 2). Of note, a significant overlap in the values of these markers was observed, and only PlGF was able to differentiate among these conditions. Next, univariate associations with subsequent pregnancy outcomes, and associations adjusted for the clinical models, are shown in Table 5 and Table 6, respectively. In the univariate analysis (Table 5), low levels of PlGF and high levels of sFlt-1 were associated with the outcomes of preeclampsia and preeclampsia + gestational hypertension. High levels of endoglin were associated with the former, but not the latter. None of the markers was able to differentiate between preeclampsia and gestational hypertension.

**Table 5. Univariate logistic model summary for angiogenic factors.**

| | PE, gHTN vs NORMAL | | | PE vs gHTN, NORMAL | | | PE vs gHTN | | | PE vs NORMAL | | |
|---|---|---|---|---|---|---|---|---|---|---|---|---|
| Effect | OR | 95% CI | p-value | OR | 95% CI | p-value | OR | 95% CI | p-value | OR | 95% CI | p-value |
| Endoglin | 1.23 | (0.99,1.52) | 0.06 | 1.31 | (1.03,1.66) | 0.025 | 1.20 | (0.80,1.79) | 0.38 | 1.29 | (1.01,1.63) | 0.038 |
| PLGF | 0.69 | (0.49,0.99) | 0.042 | 0.55 | (0.35,0.88) | 0.012 | 0.57 | (0.28,1.17) | 0.13 | 0.42 | (0.24,0.75) | 0.004 |
| sFlt1 | 1.49 | (1.06,2.08) | 0.020 | 1.69 | (1.11,2.57) | 0.014 | 1.43 | (0.72,2.87) | 0.31 | 1.64 | (1.08,2.48) | 0.02 |

| | | | | | | | | | | | | |
|---|---|---|---|---|---|---|---|---|---|---|---|---|
| PE- preeclampsia gHTN- gestational hypertension PlGF- placental growth factor sFlt-1- soluble fins-like tyrosine kinase receptor-1 | | | | | | | | | | | | |

**Table 6. Incremental effect of adding individual angiogenic markers separately to the clinical model in discrimination of hypertensive disorders of pregnancy (PE + gHTN vs. normotensive pregnancies).**

| | PE, gHTN vs NORMAL^{†} | | | PE vs gHTN, NORMAL* | | | PE vs gHTN* | | | PE vs NORMAL* | | |
|---|---|---|---|---|---|---|---|---|---|---|---|---|
| Effect | OR | 95% CI | p-value | OR | 95% CI | p-value | OR | 95% CI | p-value | OR | 95% CI | p-value |
| Endoglin | 1.19 | (0.95,1.50) | 0.15 | 1.25 | (0.94,1.67) | 0.14 | 1.12 | (0.75,1.66) | 0.55 | 1.26 | (0.94,1.68) | 0.13 |
| PLGF | 0.56 | (0.36,0.85) | 0.005 | 0.38 | (0.21,0.71) | <.001 | 0.27 | (0.08,0.91) | 0.007 | 0.40 | (0.22,0.74) | 0.002 |
| sFlt1 | 1.35 | (0.96,1.90) | 0.09 | 1.64 | (1.02,2.64) | 0.045 | 1.36 | (0.63,2.94) | 0.39 | 1.67 | (1.03,2.69) | 0.039 |

| | | | | | | | | | | | | |
|---|---|---|---|---|---|---|---|---|---|---|---|---|
| ^{†}model adjusted for parity, high risk pregnancy *model adjusted for gestations, smoking status, high risk pregnancy PE- preeclampsia gHTN- gestational hypertension PlGF- placental growth factor sFlt-1- soluble fms-like tyrosine kinase receptor-1 | | | | | | | | | | | | |

In a multivariate analysis adjusting for parity and high risk status, PlGF (p=0.005) was independently predictive of gestational hypertension + preeclampsia, while endoglin (p=0.15) and s-Flt-1 (p=0.09) were not independently predictive (Table 6). When adjusted for parity, smoking, and high risk, PlGF (p<0.001) and sFlt-1 (p=0.045) were independently predictive of preeclampsia, but endoglin was not (p=0.14). Only PlGF (p= 0.007), but neither endoglin (p-0.55) nor sFlt-1 (p=0.39), was predictive of preeclampsia versus gestational hypertension.

In the next step, a model was developed in which the incremental values of the angiogenic markers (taken together) and podocyturia were tested (Table 7). The incremental value of angiogenic markers for the prediction of preeclampsia became statistically non-significant when the incremental value of podocyturia was added to the model.

**Table 7. Incremental effects of clinical factors, all angiogenic markers taken together, and of podocyturia in prediction of preeclampsia.**

| Description | Variables | Model -2 Log Likelihood | p-value for incremental angiogenic factors effect | p-value for incremental podocyturia effect |
|---|---|---|---|---|
| gHTN +PE vs. normal ^{†} (n=267) | Clinical factors | 14.12 | --- | --- |
| | Clinical factors + all angiogenic factors | 26.49 | 0.006 | --- |
| gHTN +PE vs. normal ^{†} (n=72) | Clinical factors | 11.74 | --- | --- |
| | Clinical factors + all angiogenic factors | 25.04 | 0.004 | --- |
| | Clinical factors + all angiogenic factors + podocyturia | 46.72 | 0.11 | <.001 |
| PE vs. normal + gHTN * (n=72) | Clinical factors | 17.06 | --- | --- |
| | Clinical factors + all angiogenic factors | 29.66 | 0.006 | --- |
| | Clinical factors + all angiogenic factors + podocyturia | 73.68 | 1.00 | <.001 |
| PE vs. gHTN * (n=28) | Clinical factors | 10.64 | --- | --- |
| | Clinical factors + all angiogenic factors | 20.32 | 0.022 | --- |
| | Clinical factors + all angiogenic factors + podocyturia | 38.67 | 1.00 | <.001 |

| | | | | |
|---|---|---|---|---|
| ^{†}Clinical factors: Parity, High Risk Pregnancy *Clinical factors: Gestations, Smoking, High Risk Pregnancy PE- preeclampsia gHTN- gestational hypertension | | | | |

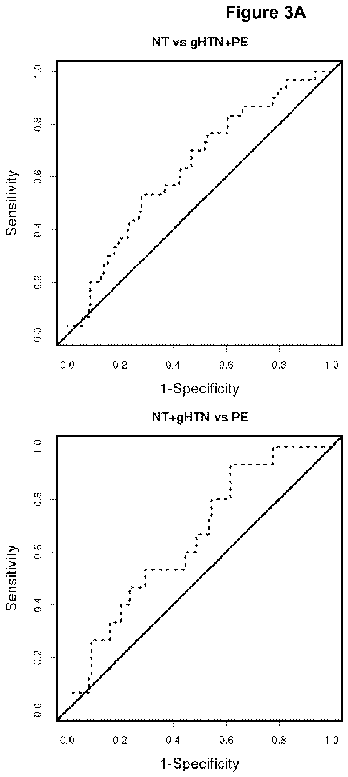

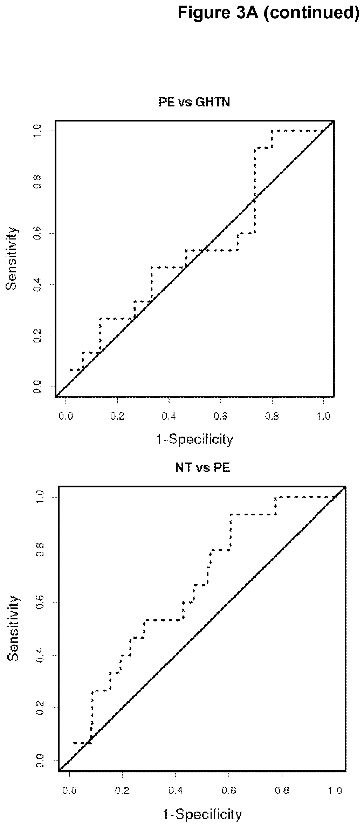

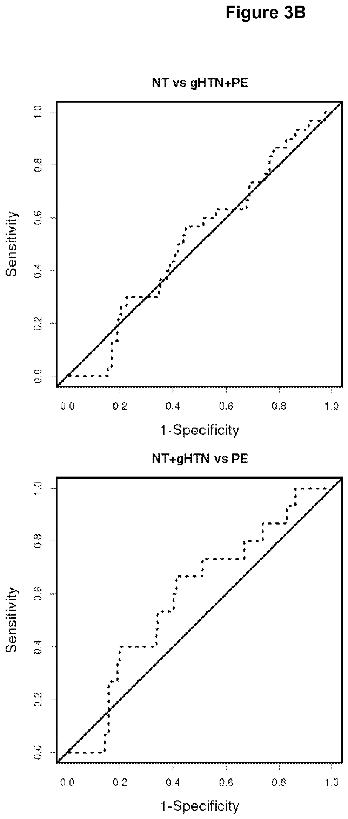

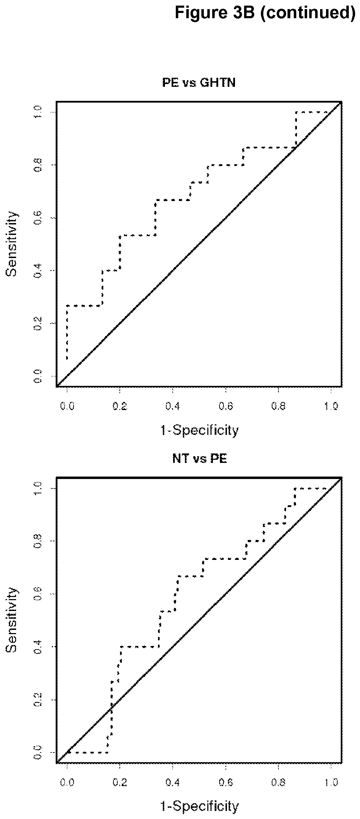

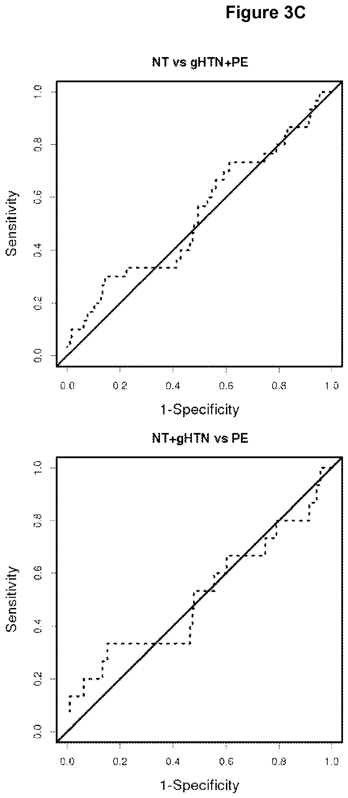

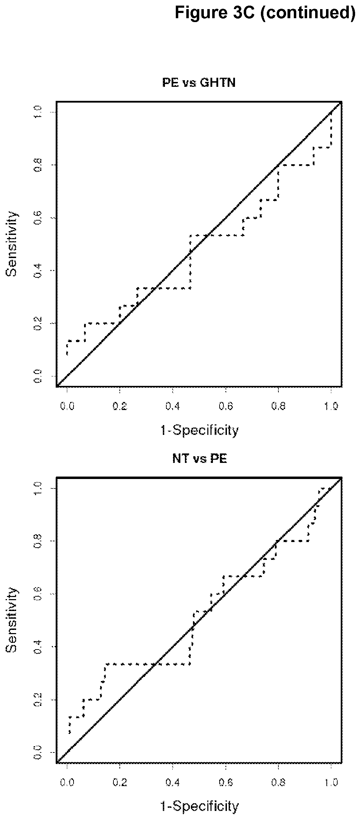

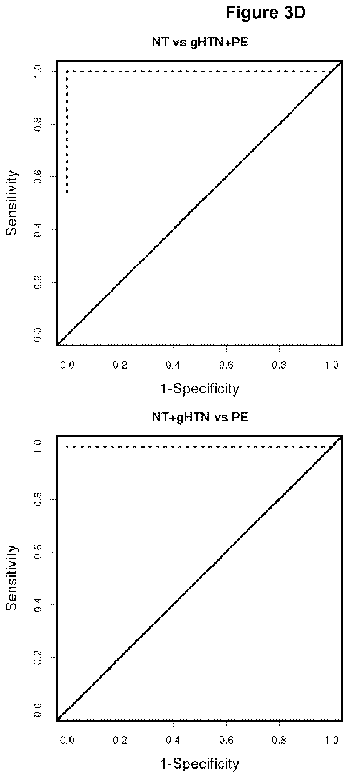

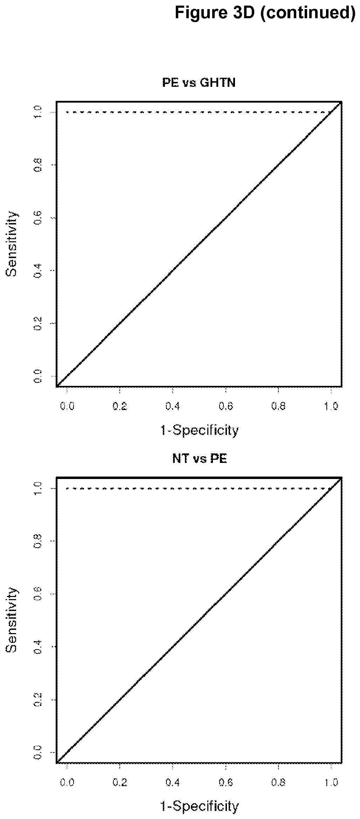

Figures 3A-3D contain graphs plotting ROC curves for sFlt-1 (3A), PIGF (3B), soluble endoglin (3C), and podocyturia (3D).

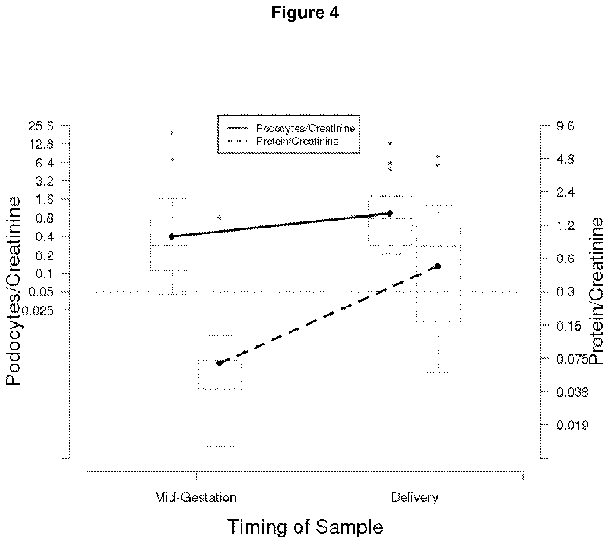

Finally, the quantitative association between podocyte count and quantitative urine protein measures was assessed by multiple regression restricted to the preeclampsia cases, with urine protein as the y-variable, and creatinine and podocyte count (ordinal scale), and gestational time period (mid-gestation or pre-delivery) as the x-variables. In this analysis, the GEE model was used to incorporate the intra-subject correlation of multiple samples per patient. The result was that creatinine (P<0.0001), the indicator of time period (pre-delivery vs. mid-gestation) (P<0.0001), and podocyte count level (P=0.04) were all independently related to protein level (Table 7). Figure 4 is a graph plotting protein per creatinine and podocytes per creatinine at mid-gestation and delivery for 15 preeclamptic patients.

These results confirm that podocyturia, i.e. urinary excretion of podocytes, is present in patients with preeclampsia, both prior to 30 weeks of gestation and at the time of delivery. As the presence of podocytes was demonstrated at a time when the diagnostic clinical features of preeclampsia, hypertension and proteinuria, were absent, these results also indicate that podocyturia can be used to identify subclinical disease and women at risk of developing preeclampsia later in pregnancy. As such, podocyturia can serve as an early marker of the disease. The presence of podocytes prior to 30 gestational weeks of pregnancy distinguished the patients who went on to develop preeclampsia from those who ultimately experienced either normotensive pregnancies or gestational hypertension only. In contrast, the angiogenic and anti-angiogenic markers measured at the same time points were unable to discriminate among specific hypertensive pregnancy disorders. A significant overlap prior to 30 weeks of gestation was observed between the patients with preeclampsia versus those with either gestational hypertension or normotensive pregnancy. Finally, a strong positive correlation exists between the number of podocytes and the degree of proteinuria, suggesting that ongoing podocyte loss can be mechanistically related to the onset and severity of proteinuria.

### Example 2 - Use of mass spectrometry to detect podocyturia in preeclampsia

### Diagnostic criteria for preeclampsia

All participating women consented before inclusion in the study. The diagnosis of preeclampsia was ascertained based on the presence of the following criteria: (a) hypertension after 20 weeks' gestation, defined as a blood pressure ≥ 140/90 mm Hg, (b) proteinuria, defined as ≥ 300 mg of protein in a 24-hour urine specimen, and/or 1+ (30 mg/L) dipstick urinalysis, in the absence of urinary tract infection, and (c) resolution of hypertension and proteinuria by 12 weeks post-partum. The diagnosis of HELLP, a severe form of preeclampsia, was confirmed by the presence of microangiopathic hemolytic anemia, elevated liver enzymes, and thrombocytopenia, as described elsewhere (Jones, Hematopathol. Mol. Hematol., 11:147-171 (1998)). Random urine samples (100-150 mL each) were obtained from 13 preeclamptic and 6 preeclamptic/HELLP syndrome consecutive patients who agreed to participate in the study (Table 8). Urine samples were used both for the podocyturia assay and LC-MS/MS technology (n=15), or for LC-MS/MS technology only (n=4). For the controls, urine was obtained from 4 normotensive consecutive pregnant women at the time of delivery.

**Table 8. Demographic and clinical data of preeclamptic (n=19) and normotensive pregnant patients (n=4) who underwent podocyturia studies.**

| | Preeclamptic pregnancies ^{a} Mean ± SD | Normotensive pregnancies Mean ± SD | P value |
|---|---|---|---|
| Maternal age (years) | 30.5 ± 7.1 | 29.7 ± 0.6 | 1 |
| Days of gestation | 235.6 ± 24.3 | 280.3 ± 8.7 | 0.0086 |
| Gravidity | 2.12 ± 1.5 | 1.33 ±0.57 | 0.3996 |
| Parity | 0.81 ± 1.12 | 0.33 ± 0.58 | 0.5379 |
| Systolic blood pressure (mm Hg) | 154.4 ± 11.35 | 118.3 ± 8.02 | 0.0085 |
| Diastolic blood pressure (mm Hg) | 93.3 ± 13.4 | 68 ± 3.46 | 0.0139 |
| Protein/creatinine ratio (grams/24 hour urine) | 3.896 ± 3.22 | 0.109 ±0.034 | 0.0086 |
| Podocyturia (number of podocytes/mg creatinine) | 3.1 ± 2.2 | absent | N/A |

| | | | |
|---|---|---|---|
| ^{a}In addition to diagnostic criteria for preeclampsia, 6 patients had laboratory abnormalities characteristic of HELLP syndrome: **h**emolysis, with a mean LDH of 350 ± 32 (normal, 122-222 U/L); **e**levated **l**iver enzymes, with a mean AST of 188 ± 102 (normal 8-43 U/L); and a **l**ow **p**latelet count, with a mean platelet count of 67 ± 34 (normal 150-450x10⁹/L). | | | |

### Podocyturia assay

Random urine samples (about 50 mL each) were centrifuged for eight minutes at 700 x g at room temperature, and processed as described elsewhere (Garovic et al., Am. J. Obstet. Gynecol., 196:320 e1-7 (2007)). Briefly, 1-mL aliquots were plated on collagen coated tissue culture slides, followed by overnight incubation at 37°C in 5% CO₂. The next day, slides were fixed with 1 mL of ice cold methanol. Each slide was incubated with a podocin antibody (Sigma, 1:200). After washing with PBS, a secondary FITC-labeled antibody was added at a dilution of 1:40 for 30 minutes. The sediment was counterstained with Hoechst nuclear stain to facilitate the differentiation of whole cells from cell fragments. Nucleated, positive staining cells were considered to be podocytes. Podocyturia was expressed as a ratio of the number of podocytes to the creatinine content of the respective urine sample, and was confirmed in the presence of ≥ 0.85 podocin-positive cells/mg creatinine (Table 8). This threshold value provided 100% sensitivity and specificity in the diagnosis of preeclampsia (Garovic et al., Am. J. Obstet. Gynecol., 196:320 e1-7 (2007)).

### LC-MS/MS

Recombinant human podocin was obtained from Novus Biologicals. The synthetic stable isotope labeled peptide, with the same sequence as the podocin tryptic peptide, was synthesized by the peptide synthesis facility at the Mayo Clinic (Rochester, MN). Random urine samples (about 50 mL each) were centrifuged for 8 minutes at 700 x g at room temperature. The supernatant was discarded, and the pellet was re-suspended in methanol fixative and stored at 4°C. Prior to digestion, the methanol fixed pellets were centrifuged at 600 x g for 10 minutes, the supernatant was removed, and the pellet was re-suspended in 50 µL of RapiGest™ SF at a concentration of 0.1% in 50 mM ammonium bicarbonate, pH 8.0. The sample was sonicated for 5 minutes, then 100 µg of trypsin was added, and the sample was sonicated again for 5 minutes. The sample was then digested in a shaking incubator set at 37°C for 4 hours. After digestion, the sample was acidified with 2 µL of formic acid and centrifuged for 10 minutes at 14,000 x g. A volume of 18 µL of patient digest was put into a well of a 96-well sample tray. A stable isotope labeled internal standard peptide was added to each sample and then analyzed by LC-MS/MS.

All samples were analyzed using a Thermo TLX-2 HPLC system, coupled to an ABSciex API 5000 triple quadrupole mass spectrometer. A 20 µL injection was made from each sample, and separations were carried out on a 100 x 3.0 mm Atlantis T3 column, with a 3 µm particle size and 120 Å pore size, run at a flow rate of 250 µL/minute. A gradient consisting of mobile phase A (100% water, 0.1% formic acid) and mobile phase B (100% acetonitrile, 0.1% formic acid) was used to resolve the peptides with a 15 minute gradient. All MS/MS conditions were optimized by infusing the synthetic stable isotope labeled podocin tryptic peptide, 39-QEAGPEPSGSGR-50 (SEQ ID NO:1). The transition that gave the best signal-to-noise was the doubly charged precursor ion to the singly charged y6 ion i.e, [M+2H⁺]⁺² → y₆⁺¹. Analyst™ software version 1.4.2 (Applied Biosystems) was used to control the instrument, acquire and process the data.

### Statistical methods

Statistical analyses were performed using JMP version 7.0.0 (SAS Institute Inc., Cary, North Carolina). All data are expressed as mean values +/- standard deviation (SD) and compared using the Wilcoxon rank sum test. For correlation studies, a two-tailed probability value of a Pearson correlation coefficient was computed. A p-value of <0.05 was pre-specified as being statistically significant.

### Tryptic Peptide from Podocin

LC-MS/MS methodology assumed a 1:1 stoichiometric ratio between the tryptic fragment isolated from podocin and the concentration of intact podocin in the sample. Adding a known amount of stable isotope labeled internal standard peptide with the same amino acid sequence as the tryptic peptide, but with a different mass, allows the mass spectrometer to distinguish between the two forms, and thus provide relative quantification.

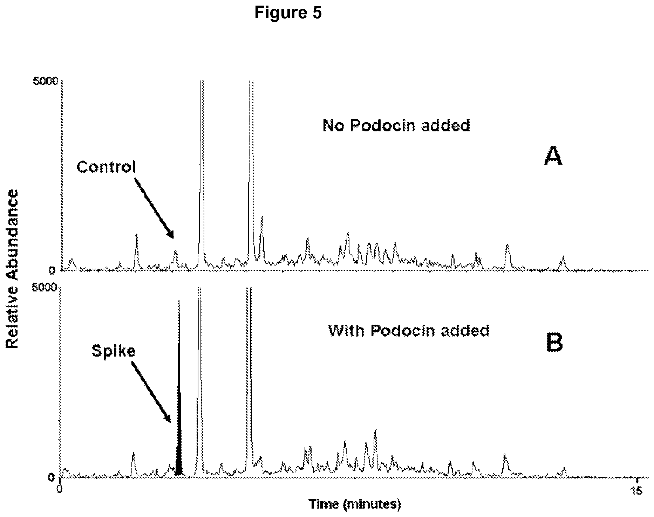

The choice of podocin in this study was based on the fact that it is highly podocyte-specific and that podocin exhibited 100% sensitivity and specificity in the diagnosis of preeclampsia. Also, its respective recombinant protein is commercially available. The tryptic peptide, QEAGPEPSGSGR, was chosen as the best peptide for quantifying podocin from urine for the following reasons: this sequence was unique to human podocin and gave the best response of all of the podocin tryptic peptides in a digest of recombinant podocin, as measured by LC-MS/MS. The identity of the peptide was confirmed by full scan MS/MS and by standard addition of recombinant podocin digests to the frozen cell digests. The reproducibility of the digestion protocol was examined using frozen mouse podocytes as a surrogate matrix. Conditionally immortalized mouse podocytes were obtained from Dr. Peter Mundel. In this experiment, differentiated podocytes were used, after differentiation by changing from permissive to non-permissive conditions, as described elsewhere (Mundel et al., Exp. Cell Res., 236:248-258 (1997)). All digests contained the same volume of frozen mouse podocytes that were thawed and solubilized in RapiGest™ buffer. LC-MS/MS chromatograms specific to the human podocin tryptic peptide, QEAGPEPSGSGR, digested in the mouse podocyte matrix were obtained (Figures 5A and 5B). The chromatogram in Figure 5A shows the control mouse podocyte digest without recombinant human podocin added prior to digestion, and Figure 5B shows the same mouse podocytes spiked with recombinant human podocin prior to digestion. Figure 5B clearly shows the highlighted peak representing the tryptic peptide, QEAGPEPSGSGR, from the recombinant human podocin spiked into the mouse podocytes. The response for this peptide was at the same retention time as the internal standard added to the sample after digestion, and before injection (chromatogram not shown). Table 9 shows the peak areas for the tryptic peptide and the internal standard for each of the three replicates, along with their respective coefficient of variation values. These results indicate that the method is reproducible using the frozen mouse podocyte matrix.

**Table 9. Peak areas for the tryptic peptide and the internal standard for each of the three replicates of the control mouse podocyte digest spiked with the recombinant human podocin.**

| Sample name | Analyte Peak Area (counts) | Internal Standard Peak Area (counts) | *Area ratio* |
|---|---|---|---|
| Mouse podocyte digest Spike 1 | 3290 | 159000 | *0.021* |
| Mouse podocyte digest Spike 2 | 2890 | 153000 | *0.019* |
| Mouse podocyte digest Spike 3 | 3210 | 155000 | *0.021* |
| Average (mean) | 3130 | 155667 | *0.020* |
| Standard deviation | 212 | 3055 | *0.013* |
| *% Coefficient of variation* | *7* | *2* | *5* |

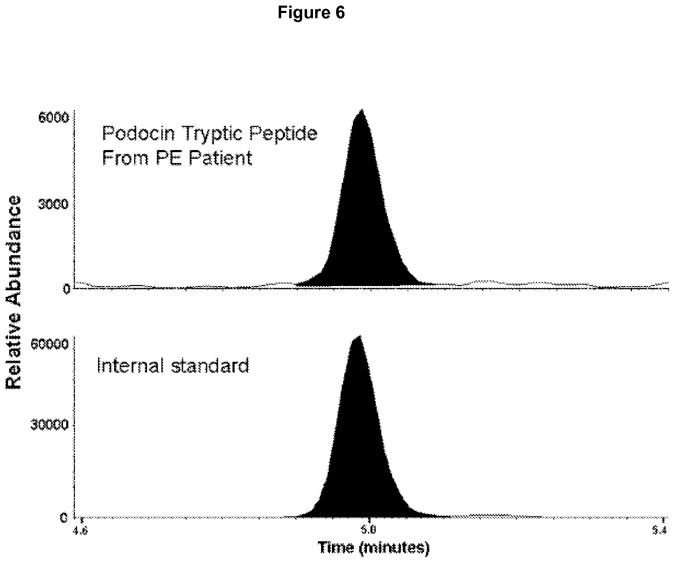

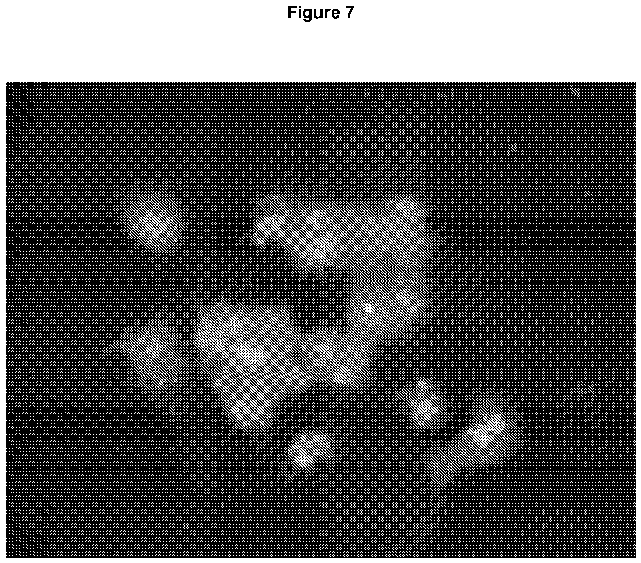

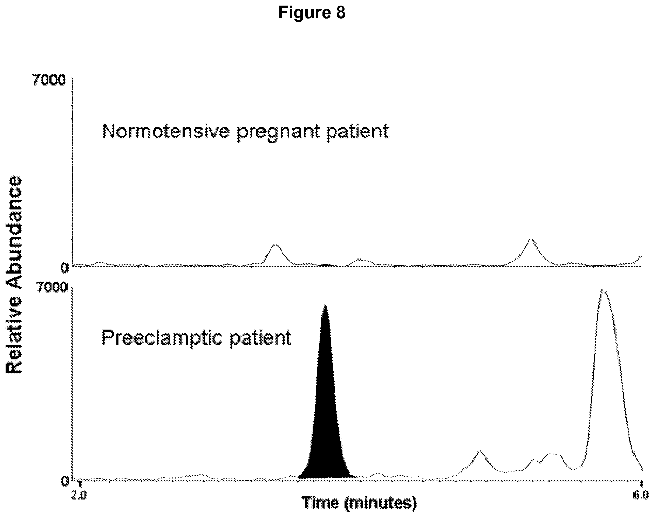

All 15 urine samples obtained from preeclamptic patients demonstrated the podocin tryptic peptide of interest. Figure 6 shows an LC-MS/MS chromatogram representative of the results found using cells from a patient classified as having preeclampsia: the highlighted podocin tryptic peptide peak from the preeclampsia patient on the top, and the peak from the internal standard on the bottom. Figure 6 demonstrated that the tryptic peptide derived from podocin in the patient sample has the same retention time as the synthetic stable isotope labeled internal standard peptide. In preeclamptic women, a positive correlation was present between the quantity of the podocin peptide in their urines, and both the amount of proteinuria in the respective urine samples (p=0.03), and their systolic blood pressures at the time of urine collection (p=0.001). The presence of podocytes in the respective urine samples at the time of delivery was further confirmed by the overnight podocyte culture method (Figure 7), indicating the presence of ≥ 0.85 podocin-positive cells/mg creatinine in all tested samples. At the 6-week postpartum visit, podocyturia was absent in all women in whom it was present at the time of delivery. In the final step, urinary samples were collected from 4 patients with normotensive pregnancies and 4 patients with preeclampsia, and each whole urine sample was used for the detection and quantification of the podocyte tryptic peptide of interest. The quantification of the podocin peptide was performed by a single point calibration, using a known amount of the internal standard (expressed in fmol units) that was added to each sample, and expressed in fmol of podocin/mg of creatinine in the respective samples. Podocin tryptic peptide from fixed cells taken from normotensive pregnant patients' urine samples, as compared to preeclampsia patients' urine samples, showed a significantly lower response : 0.4 ± 0.04 vs. 4.6 ± 2.3 fmol/mg creatinine, respectively, p=0.01 (Figure 8). The reproducibility of fixed cell digests was evaluated by using solubilized fixed cells from a single patient, splitting the sample three ways, then performing the digestion and LC-MS/MS analysis. The results, presented in Table 10, demonstrated that the method was reproducible for fixed cells derived from patient urine.

**Table 10. Peak areas for the tryptic peptide for each of the three replicates of the fixed cells derived from the urine of a preeclamptic patient.**

| Sample name | Analyte Peak Area (counts) | Internal Standard Peak Area (counts) | Area ratio |
|---|---|---|---|
| Patient Fixed cell Digest 1 | 921 | 220000 | 4.2E-03 |
| Patient Fixed cell Digest 2 | 777 | 173000 | 4.5E-03 |
| Patient Fixed cell Digest 3 | 947 | 201000 | 4.7E-03 |
| Average (mean) | 882 | 198000 | 4.46E-03 |
| Standard deviation | 92 | 23643 | 0.25E-03 |
| % Coefficient of variation | 10 | 12 | 6 |

### Example 3 - Use of flow cytometry to detect podocyturia in preeclampsia

An operator-independent and highly reproducible method for using flow cytometry to detect podocyturia in preeclampsia was developed.

The diagnosis of preeclampsia was confirmed in the presence of hypertension (>140/90 mm Hg) and proteinuria >0.3 g/24 hour urine. The diagnosis of HELLP was confirmed based on the accepted clinical criteria of Hemolysis, Elevated Liver enzymes, and Low Platelet count. Random urine samples within 24 hours prior to delivery were collected and centrifuged. The cell pellets were suspended in FACS buffer, and cells were stained for a podocyte-specific protein, podocin (anti-NPHS2-Podocin-Aviva Systems Biology). Cells were washed twice with FACS buffer and stained with a secondary antibody in FACS buffer for 30 minutes. Then, the cells were washed twice and fixed in paraformaldehyde. Flow cytometry data were acquired using a Becton Dickinson (BD, Franklin Lakes, NJ, USA) FACS Calibur and analyzed using Flowjo software (FlowJo, Ashland, OR, USA). Podocin-positive cells were considered to be podocytes. Podocyturia was expressed as a ratio of the number of podocytes (mean ± standard deviation) to the creatinine content of the respective urine sample.

Four patients in each of the following groups were analyzed: preeclampsia, HELLP syndrome, and normotensive controls. Patients with preeclampsia had significantly higher proteinuria (2.9 ± 2 grams/24 hours) than either those with HELLP syndrome (0.29 ± 0.93 grams/24 hours), or normotensive controls (0.1 ± 0.06 grams/24 hours). The degree of podocyturia was higher in patients with preeclampsia (160 ± 87 cells/mg creatinine) than in those with either normotensive pregnancies (22 ± 20, p=0.02) or HELLP syndrome (21.5 ± 8.6, p=0.02). No difference was observed between normotensive vs. HELLP pregnancies (p=0.5). There was a positive correlation between the degree of proteinuria and podocyturia, for the group as a whole (r= 0.85, p<0.001).

These results demonstrate that flow cytometry techniques can be designed to detect podocyturia. In addition, the absence of podocyturia in patients with HELLP syndrome, with either absent or minimal proteinuria, further supports its distinct pathophysiology with respect to preeclampsia.

### Example 4 - Antibodies for an ELISA designed to detect podocyturia in preeclampsia

Antibodies for an operator-independent and highly reproducible method for using ELISA to detect podocyturia in preeclampsia were developed. An N-terminal polypeptide fragment of human podocin was selected to have the following amino acid sequence: GRGRQEAGPEPSGSGRAGTPGC (SEQ ID NO:2). This polypeptide fragment was termed hpod 35-55 and was used to generate anti-podocin antibodies.

A C-terminal polypeptide fragment of human podocin was selected to have the following amino acid sequence: CGSLPFPSPSKPVEPLNPKKKDSPML (SEQ ID NO:3). This polypeptide fragment was termed hpod 359-383 and was used to generate anti-podocin antibodies.

### Example 5 - Use of reverse transcriptase-polymerase chain reaction (RT-PCR) assays to detect podocyturia in preeclampsia

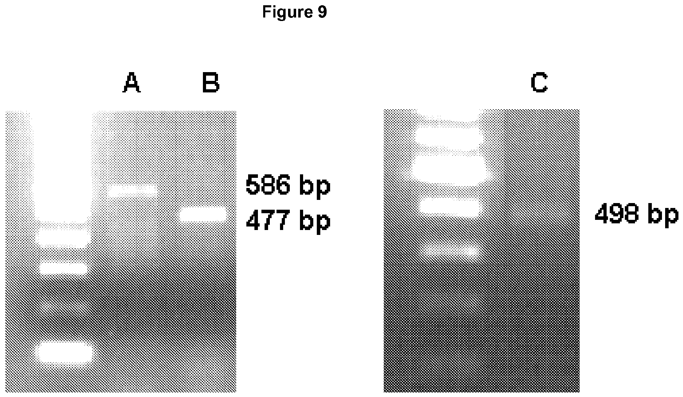

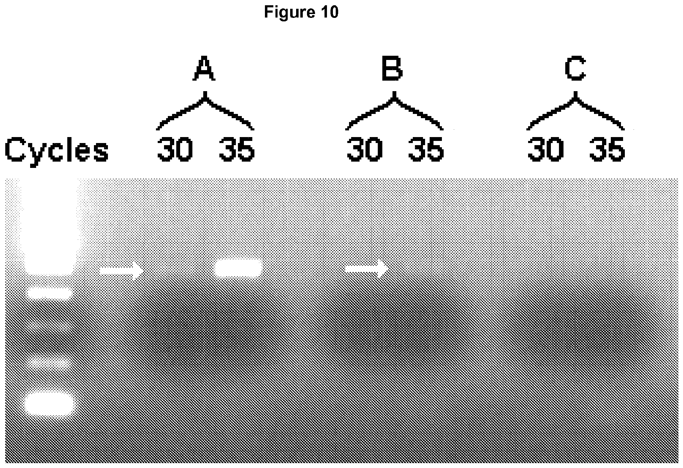

Urine samples from preeclamptic and normotensive pregnant women were obtained and individually centrifuged to yield cellular pellets from which total RNA was isolated using a Qiagen RNeasy kit. RT-PCR was performed using primers specific for synaptopodin, podocin, and nephrin. Clear bands were obtained corresponding with the predicted product sizes (586, 477, and 498 bp, respectively) for a urine sample obtained from a preeclamptic pregnant women (Figure 9). In addition, mRNA for synaptopodin, podocin, and nephrin was present in urine samples from pregnant women with preeclampsia (Figure 9 and Figure 10, lane A). In contrast, podocin mRNA was either weakly present (Figure 10, lane B) or undetectable (Figure 10, lane C) in urine samples from normotensive pregnant controls after 35 cycles of amplification.

These results demonstrate that RT-PCR assays (e.g., podocin RT-PCR assays) can be designed to detect podocyturia.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A flow cytometry method for the early detection of a pregnant woman susceptible to developing preeclampsia, wherein said method comprises:
(a) performing flow cytometry using a urine sample obtained from a pregnant woman, prior to development of preeclampsia and from gestational week 23 to gestational week 28, to detect the presence of an elevated level of urinary podocytes with an anti-podocin antibody, and
(b) classifying said pregnant woman as being susceptible to developing preeclampsia later during her pregnancy.

2. The method of claim 1, wherein said urine sample was obtained from said pregnant woman from gestational week 24 to gestational week 28.

3. The method of claim 1, wherein said urine sample was obtained from said pregnant woman from gestational week 25 to gestational week 28.

4. The method of claim 1, wherein said urine sample was obtained from said pregnant woman from gestational week 26 to gestational week 28.

5. The method of claim 1, wherein said urine sample was obtained from said pregnant woman from gestational week 23 to gestational week 27.

6. The method of claim 1, wherein said urine sample was obtained from said pregnant woman from gestational week 23 to gestational week 26.

7. An enzyme-linked immunosorbent assay method for the early detection of a pregnant woman susceptible to developing preeclampsia wherein said method comprises:
(a) performing an enzyme-linked immunosorbent assay using a urine sample obtained from a pregnant woman, prior to development of preeclampsia and from gestational week 23 to gestational week 28, to detect the presence of an elevated level of urinary podocytes with an anti-podocin antibody, and
(b) classifying said pregnant woman as being susceptible to developing preeclampsia later during her pregnancy.

8. The method of claim 7, wherein said urine sample was obtained from said pregnant woman from gestational week 24 to gestational week 28, or wherein said urine sample was obtained from said pregnant woman from gestational week 25 to gestational week 28, or wherein said urine sample was obtained from said pregnant woman from gestational week 26 to gestational week 28, or wherein said urine sample was obtained from said pregnant woman from gestational week 23 to gestational week 27, or wherein said urine sample was obtained from said pregnant woman from gestational week 23 to gestational week 26.

## Patentansprüche

1. Durchflusszytometrieverfahren für den frühen Nachweis der Anfälligkeit einer Schwangeren für die Entwicklung von Präeklampsie, wobei das Verfahren Folgendes umfasst:
(a) Durchführen von Durchflusszytometrie unter Verwendung einer von einer Schwangeren vor der Entwicklung von Präeklampsie und von Schwangerschaftswoche 23 bis Schwangerschaftswoche 28 erhaltenen Urinprobe zum Nachweisen des Vorliegens eines erhöhten Spiegels von Urin-Podozyten mit einem Anti-Podocin-Antikörper und
(b) Klassifizieren der Schwangeren als anfällig für die Entwicklung von Präeklampsie im späteren Verlauf ihrer Schwangerschaft.

2. Verfahren nach Anspruch 1, wobei die Urinprobe von der Schwangeren von Schwangerschaftswoche 24 bis Schwangerschaftswoche 28 erhalten wurde.

3. Verfahren nach Anspruch 1, wobei die Urinprobe von der Schwangeren von Schwangerschaftswoche 25 bis Schwangerschaftswoche 28 erhalten wurde.

4. Verfahren nach Anspruch 1, wobei die Urinprobe von der Schwangeren von Schwangerschaftswoche 26 bis Schwangerschaftswoche 28 erhalten wurde.

5. Verfahren nach Anspruch 1, wobei die Urinprobe von der Schwangeren von Schwangerschaftswoche 23 bis Schwangerschaftswoche 27 erhalten wurde.

6. Verfahren nach Anspruch 1, wobei die Urinprobe von der Schwangeren von Schwangerschaftswoche 23 bis Schwangerschaftswoche 26 erhalten wurde.

7. ELISA(Enzyme-linked Immunosorbent Assay)-Verfahren für den frühen Nachweis der Anfälligkeit einer Schwangeren für die Entwicklung von Präeklampsie, wobei das Verfahren Folgendes umfasst:
(a) Durchführen eines ELISA-Tests unter Verwendung einer von einer Schwangeren vor der Entwicklung von Präeklampsie und von Schwangerschaftswoche 23 bis Schwangerschaftswoche 28 erhaltenen Urinprobe zum Nachweisen des Vorliegens eines erhöhten Spiegels von Urin-Podozyten mit einem Anti-Podocin-Antikörper und
(b) Klassifizieren der Schwangeren als anfällig für die Entwicklung von Präeklampsie im späteren Verlauf ihrer Schwangerschaft.

8. Verfahren nach Anspruch 7, wobei die Urinprobe von der Schwangeren von Schwangerschaftswoche 24 bis Schwangerschaftswoche 28 erhalten wurde oder wobei die Urinprobe von der Schwangeren von Schwangerschaftswoche 25 bis Schwangerschaftswoche 28 erhalten wurde oder wobei die Urinprobe von der Schwangeren von Schwangerschaftswoche 26 bis Schwangerschaftswoche 28 erhalten wurde oder wobei die Urinprobe von der Schwangeren von Schwangerschaftswoche 23 bis Schwangerschaftswoche 27 erhalten wurde oder wobei die Urinprobe von der Schwangeren von Schwangerschaftswoche 23 bis Schwangerschaftswoche 26 erhalten wurde.

## Revendications

1. Méthode de cytométrie en flux pour la détection précoce du risque de développement de pré-éclampsie chez une femme enceinte, où ladite méthode comprend :
(a) la mise en oeuvre d'une cytométrie en flux sur un échantillon d'urine obtenu auprès d'une femme enceinte, avant le développement d'une pré-éclampsie et entre la semaine de gestation 23 et la semaine de gestation 28, pour détecter la présence d'un niveau supérieur de podocytes urinaires à l'aide d'un anticorps anti-podocine, et
(b) la classification de ladite femme enceinte comme risquant de développer une pré-éclampsie à un stade ultérieur de sa grossesse.

2. Méthode selon la revendication 1, où ledit échantillon d'urine est obtenu auprès de ladite femme enceinte entre la semaine de gestation 24 et la semaine de gestation 28.

3. Méthode selon la revendication 1, où ledit échantillon d'urine est obtenu auprès de ladite femme enceinte entre la semaine de gestation 25 et la semaine de gestation 28.

4. Méthode selon la revendication 1, où ledit échantillon d'urine est obtenu auprès de ladite femme enceinte entre la semaine de gestation 26 et la semaine de gestation 28.

5. Méthode selon la revendication 1, où ledit échantillon d'urine est obtenu auprès de ladite femme enceinte entre la semaine de gestation 23 et la semaine de gestation 27.

6. Méthode selon la revendication 1, où ledit échantillon d'urine est obtenu auprès de ladite femme enceinte entre la semaine de gestation 23 et la semaine de gestation 26.

7. Méthode de dosage immunologique ELISA pour la détection précoce du risque de développement de pré-éclampsie chez une femme enceinte, où ladite méthode comprend :
(a) la mise en oeuvre d'un dosage immunologique ELISA sur un échantillon d'urine obtenu auprès d'une femme enceinte, avant le développement d'une pré-éclampsie et entre la semaine de gestation 23 et la semaine de gestation 28, pour détecter la présence d'un niveau supérieur de podocytes urinaires à l'aide d'un anticorps anti-podocine, et
(b) la classification de ladite femme enceinte comme risquant de développer une pré-éclampsie à un stade ultérieur de sa grossesse.

8. Méthode selon la revendication 7, où ledit échantillon d'urine est obtenu auprès de ladite femme enceinte entre la semaine de gestation 24 et la semaine de gestation 28, ou où ledit échantillon d'urine est obtenu auprès de ladite femme enceinte entre la semaine de gestation 25 et la semaine de gestation 28, ou où ledit échantillon d'urine est obtenu auprès de ladite femme enceinte entre la semaine de gestation 26 et la semaine de gestation 28, ou où ledit échantillon d'urine est obtenu auprès de ladite femme enceinte entre la semaine de gestation 23 et la semaine de gestation 27, ou où ledit échantillon d'urine est obtenu auprès de ladite femme enceinte entre la semaine de gestation 23 et la semaine de gestation 26.
